# EUROPÄISCHE PATENTSCHRIFT

(11) **EP 1 183 513 B1**
(45) Veröffentlichungstag und Bekanntmachung des Hinweises auf die Patenterteilung: **21.07.2004**
(21) Anmeldenummer: 00929566.8
(22) Anmeldetag: 30.05.2000
(51) Int. Cl.: G01N 1/12

(54) **VORRICHTUNG ZUR ENTNAHME VON SCHLACKENPROBEN**
DEVICE FOR TAKING SLAG SAMPLES
DISPOSITIF POUR LE PRELEVEMENT D'ECHANTILLONS DE LAITIER

(30) Priorität: 01.06.1999 DE 29909595 U
(43) Veröffentlichungstag der Anmeldung: 06.03.2002
(73) Patentinhaber: Minkon Sampler-Technik GmbH Probennahme Aus Metallschmelzen, 40689 Erkrath (DE)
(72) Erfinder: WÜNSCH, Hartmut, D-40822 Mettmann (DE); WÜNSCH, Gerhard, D-40822 Mettmann (DE)
(74) Vertreter: König, Reimar
(86) Internationale Anmeldenummer: PCT/EP2000/004937
(87) Internationale Veröffentlichungsnummer: WO 2000/073765

(56) Entgegenhaltungen:
- DE-A- 19 752 743
- SU-A- 646 215
- US-A- 3 877 309
- US-A- 4 102 197
- US-A- 4 557 152
- PATENT ABSTRACTS OF JAPAN vol. 011, no. 129 (P-570), 23. April 1987 (1987-04-23) & JP 61 271452 A (KAWASOU DENKI KOGYO KK), 1. Dezember 1986 (1986-12-01)

## Beschreibung

Die Erfindung betrifft eine Vorrichtung zur Entnahme von Schlackenproben aus eisen- oder stahlerzeugenden Anlagen und nimmt die Priorität des deutschen Gebrauchsmusters 299 09 595.9 in Anspruch, auf die inhaltlich Bezug genommen wird.

Zur Entnahme von Roheisenschlackenproben aus beispielsweise Rinne, Fuchs oder Pfanne oder zur Entnahme von Stahlschlackenproben aus einem Konverter, Pfanne, ESU-Anlage oder Behandlungsanlagen sind verschiedene Verfahren bekannt, die in der Regel zunächst zu Proben führen, die, um für eine spektrometrische Analyse oder Röntgenfluoreszenzanalyse verwendbar zu sein, in einem aufwendigen Prozeß zu einer talerförmigen Schlackenprobe verarbeitet werden müssen.

So wird nach einem bekannten Verfahren zur Schlackenprobenentnahme zunächst eine Stahlstange oder ein ähnlicher Metallgegenstand in die Schmelze eingetaucht. Dazu können einfache Stahlrohre, Stahlstangen oder auch aufwendiger gestaltete Elemente größerer Oberfläche, die außen an einer Tauchproben- oder Tauchtemperatursonde befestigt sein können, verwendet werden. Beim Herausziehen des Metallgegenstandes aus der Schmelze bleibt die Schlacke an der Metalloberfläche haften. Zur Vorbereitung der Schlackenprobe für die Analyse muß die Schlacke von dem Metallgegenstand abgeschlagen und die einzelnen Schlackenstücke zu einer talerförmigen Probe weiterverarbeitet werden.

Die Verarbeitung der Schlacke kann teilweise bis zu einer Stunde dauern und macht dieses Probeentnahmeverfahren zeit- und kostenintensiv. Darüber hinaus ist durch die zeitverzögerte Verfügbarkeit der Analyseergebnisse eine direkte Steuerung des Schmelzprozesses nicht möglich. Neben dem hohen Verarbeitungsaufwand können auch Ergebnisverfälschungen bei der Analyse aufgrund unreiner Proben bei mehrfacher Verwendung und dadurch bedingte Kontamination der Entnahmesonden auftreten. In der Regel wird zwar die Restschlacke der vorhergehenden Probenentnahme gründlich von der Oberfläche der Entnahmesonde entfernt, doch sind in der Praxis Meßwertverfälschungen aufgrund von Schlackeresten kaum zu vermeiden.

Weitere Meßwertverfälschungen sind auf Zusatzstoffe wie Bindemittel zurückzuführen, die bei der Aufbereitung der Schlackenprobe für die Analyse in die Probe eingebracht werden müssen.

Eine entsprechende Probenentnahme ist in der US-Patentschrift 5 435 196 beschrieben.

Die beschriebene, weit verbreitete Verfahrensweise liefert somit noch keine befriedigenden Ergebnisse. Bei einem neuen Verfahren wird die Schlackenprobe durch Absaugen der Schlacke von der Schmelzenoberfläche mit Hilfe einer Saugsonde gewonnen. Die Sonde entspricht in ihrer Bauweise im wesentlichen den zur Probenentnahme aus der Stahlschmelze eingesetzten Sonden.

Die Sonde kann jedoch nicht, anders als bei der Stahlprobenentnahme, in die Schmelze eingetaucht werden, sondern muß mit ihrer Einlauföffnung präzise in der Schlackenschicht positioniert werden. Dies bereitet bei dünnen Schlackenschichten erhebliche Schwierigkeiten, da sich eine Kontamination der Probe mit flüssigem Metall nur schwer vermeiden läßt. Auch bei bestimmten metallurgischen Gefäßen, wie beispielsweise Konvertern oder Behandlungsanlagen, ist eine Probenentnahme mit Hilfe der Saugsonde aufgrund von Positionierungsschwierigkeiten kaum möglich. Wird die Sonde zu tief eingetaucht, so daß Metallschmelze mit in die Probekokille gelangt, ist die Schlackenprobe für die Analyse unbrauchbar.

Die entnommenen Schlackenproben sind i.d.R. spröde und neigen zur Rißbildung, was zum Brechen der Proben führen kann. Für die spektrometrische Analyse läßt sich die Probe dann nicht mehr verwenden.

Bei den im Bereich der Stahlprobenentnahme bekannten Sonden besteht das Problem der Eintauchtiefe nicht, da die Stahlprobensonde beliebig tief in die Schmelze eingetaucht werden kann, um eine Stahlprobe zu entnehmen, ohne daß die Gefahr besteht, daß, wie bei der Schlackenprobenentnahme, eine Kontamination der Probe aufgrund einer Fehlpositionierung der Entnahmesonde zu befürchten ist. Die Erkenntnisse und Lösungen aus diesem Bereich lassen sich folglich nicht auf den Schlackenentnahmebereich übertragen.

Selbst bei feinfühliger Positionierung einer Stahlprobensonde in der Schlackenschicht läßt sich eine reine Schlackenprobe kaum erreichen. Insbesondere ist es in diesem Anwendungsbereich Ziel, den Eintritt von Schlacke zu vermeiden.

Daher sind Stahlprobensonden in der Regel so konstruiert, daß sie gerade den Eintritt von Schlacke verhindern.

Aus JP-61271452 ist weiter eine Vorrichtung zur Entnahme von Schlackenproben, die einen Probenraum aufweist, bekannt. Die Schlacke wird beim Eintauchen der Vorrichtung in die Schmelze durch einen Einlauf nach oben in den Probenraum geführt.

Der Erfindung liegt somit das Problem zugrunde, eine Entnahmevorrichtung zu schaffen, die eine einfache und sichere Probenentnahme von Schlacken erlaubt.

Das Problem wird gelöst durch eine Vorrichtung zur Entnahme von Schlackenproben nach dem unabhängigen Anspruch.

Der Querschnitt der Einlauföffnung kann in mindestens einem Bereich im Verhältnis zum Probenraumquerschnitt gering sein, während der vorzugsweise ringförmige Probenraum eine geringe Höhe im Verhältnis zu seinem Querschnitt aufweisen kann.

Beim Eintauchen der Probenentnahmevorrichtung in die Schlackenschicht füllt sich die Probenkammer durch die unterhalb der Probenkammer liegende Einlauföffnung (Kokilleneinlauf) mit Schlacke. Eine Kontamination durch Schmelze ist nun bereits dadurch ausgeschlossen, daß die Schlacke aufgrund ihres in bezug auf die Schmelze geringeren spezifischen Gewichtes immer zuerst in die Probenkammer eintritt. Vorzugsweise ist die Probenkammer bezüglich ihres Volumens so ausgestaltet, daß sie in jedem Falle unabhängig von der Eintauchtiefe der Probensonde vollständig nur mit Schlacke gefüllt wird.

Der in Bezug auf den Probenraum vorzugsweise enger ausgestaltete Speiser führt zu einer sauberen Trennebene zwischen Schlacke und Stahl, selbst wenn eine Schlackenprobe von einer lediglich dünnen Schlackenschicht entnommen wird und Stahl mit in den Einlauf gelangt.

Der Einlaufbereich vor dem Speiser kann trichterförmig ausgebildet sein, wobei die Dimensionierung des Trichters so gewählt sein kann, daß selbst bei sehr dünnen Schlackenschichten eine sichere stahlfreie Schlackenprobenentnahme möglich ist. Unter solchen Bedingungen wird ein breiterer Trichterdurchmesser gewählt, so daß ein Zugriff auf eine größere Schlackenfläche möglich ist, die ihren Weg dann über den Einlauftrichter in die Probenkammer mit relativ geringem Durchmesser findet, so daß die Probenkammer trotz der dünnen Schlackenschicht vollständig von Schlacke gefüllt wird. Dabei ist das Volumen des Einlauftrichters vorzugsweise größer als das Volumen der Probenkokille.

Ferner kann der Probenraum durch eine Metallplatte begrenzt sein, die eine Wandstärke von weniger als 2 mm aufweisen kann. Diese geringe Wandstärke der Metallplatte vermeidet aufgrund der geringeren Wärmeleitung ein zu schnelles Abkühlen der Schlackenoberfläche, wodurch ein Reißen der Schlackenprobe verhindert wird.

Mit der auf der Metallplatte entstandenen Probenoberfläche ist eine direkte Analyse der Schlackenprobe ohne oder mit nur geringfügiger Bearbeitung möglich. Zur weiteren Erleichterung der Probenanalyse kann im Schlackenraum ein Probenring ausgebildet sein, der die Probe beim Transport und während der Analyse umschließt, stabilisiert und dem Probenraum zusammen mit der Schlackenprobe entnommen wird. Insbesondere beim Entfernen der Probe aus dem Probenraum wird so verhindert, daß die Probe durch mechanische Einwirkungen zerbricht. Darüber hinaus ermöglicht der Probenring eine exakte Fixierung der Probe in der Aufnahmemimik der Analyseautomaten.

Für eine Probenentnahme wird die Sonde durch die Schlacke durchgetaucht, woraufhin sich die Probenkokille aufgrund des ferrostatischen Drucks füllt. Reicht dieser nicht aus, kann die Sonde weiter eingetaucht werden. Das Füllen der Probenkokille kann durch Erzeugen eines Unterdrucks unterstützt werden.

Die Probeneinfassung kann unterbrochen sein, um eine Trennung von Probeneinfassung und Probe - falls erforderlich - zu erleichtern. Vorzugsweise verbleibt die Probe aber, wie oben beschrieben, in der Probeneinfassung. Die Probeneinfassung kann darüber hinaus Einkerbungen oder Riefen aufweisen, um die Haftung der Probe in der Einfassung zu vergrößern und dadurch ein Herausfallen der Probe zu verhindern.

Bei der erfindungsgemäßen Lösung wird die Schlacke im Einlauf zunächst höher geführt als der Einlauf der Probenkokille. Vom höchsten Punkt läuft die Schlacke unter Wirkung der Schwerkraft - evtl. verstärkt durch den ferrostatischen Druck - in die Probenform. Die Einlauföffnung des Probenraums - und damit auch der Probenraum selbst - kann dabei unterhalb einer Überlaufkammer angeordnet sein. Diese Überlaufkammer wird über den eigentlichen Sondeneinlauf gespeist. Der Überlauf kann auch als gebogenes Einlaufrohr ausgebildet sein. Ein Rücklaufen der flüssigen Schlacke aus dem Probenraum in den Einlauf ist bei dieser Anordnung nicht möglich. Somit stellt diese Anordnung eine auslaufsichere Schlackensonde dar, die besonders bei niedrig viskosen Schlacken eine sichere und kontaminationsfreie Probenentnahme ermöglicht.

Im folgenden wird die Erfindung anhand eines in der Zeichnung dargestellten Ausführungsbeispiels des näheren erläutert.

In der Zeichnung zeigen:
Fig. 1 eine nicht erfindungsgemäße, als Beispiel dargestellte, Vorrichtung zur Probenentnahme von Schlackenproben mit einem unterhalb einer Metallplatte angeordneten Probeneinlauf;
Fig. 2 eine schematische Darstellung einer Probenentnahme mit einer Vorrichtung gemäß der Ausführungsform der Fig. 1.
Fig. 3 eine Kombisonde gemäß der Ausführungsform der Fig. 1.
Fig. 4 eine erfindungsgemäße Vorrichtung mit einer Überlaufkammer und einem unterhalb der Kammer angeordneten Probenraum.
Fig. 5 eine Kombisonde gemäß der Ausführungsform der Fig. 4.

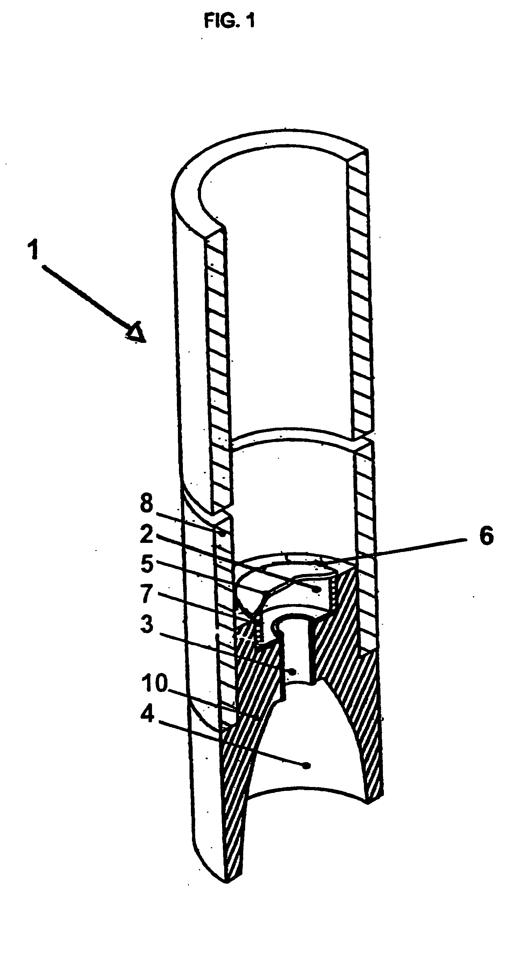

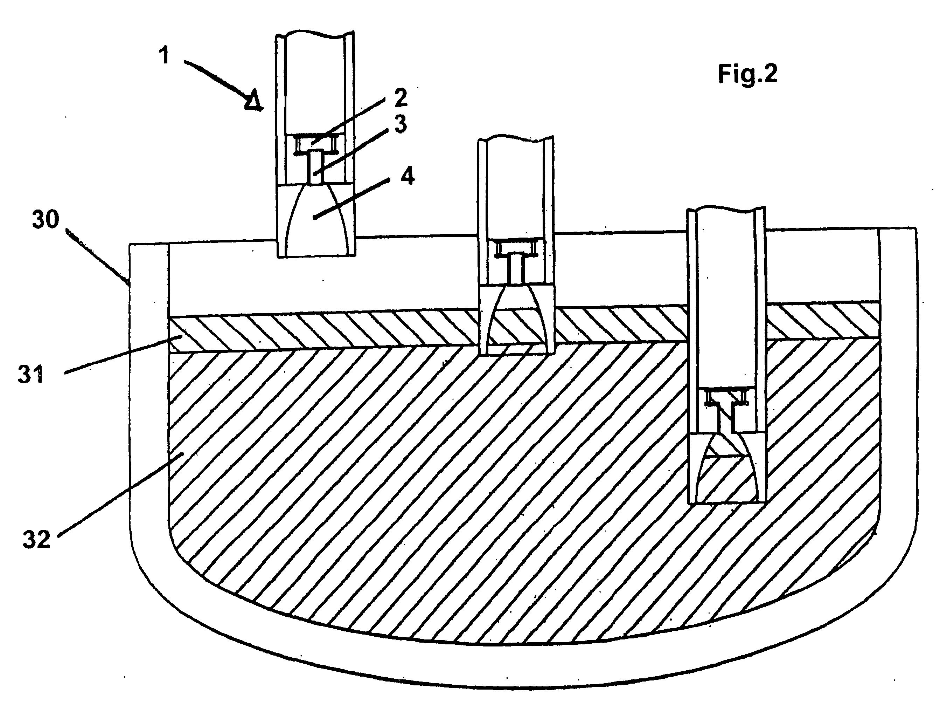

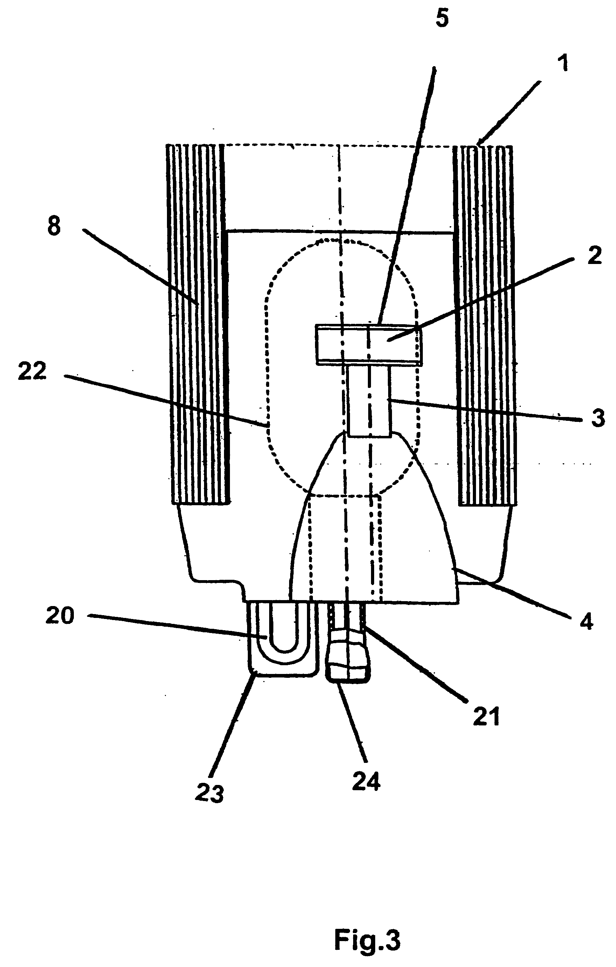

Die Vorrichtung zur Schlackenprobenentnahme besteht aus einer im Querschnitt kreisförmigen Sonde 1 mit einem Probenraum 2, einem Speiser 3 und einem Einlauftrichter 4. Der Speiser besteht aus einem Steigrohr und einem Kokilleneinlauf. Der Probenraum 2 ist bei der Ausführungsform der Fig. 1 nach oben, d.h. in Richtung der Sonde durch eine metallische Abkühlplatte 5 begrenzt. Die Probenentnahmeeinheit 2, 3, 4, 5 befindet sich im unteren, d.h. der Schlacke zugewandten Bereich der Sonde 1, die in ihrem oberen Bereich aus einem Papprohr 8 besteht.

Der unterhalb der Abkühlplatte 5 angeordnete Probenraum 2 steht über den Speiser 3 mit dem Einlauftrichter 4 in Verbindung. Der Speiser 3 weist einen in bezug auf den Querschnitt des Probenraumes und des Einlauftrichters geringen Querschnitt auf.

Der Probenraum 2 ist von einem Probenring 7 umgeben, der die Schlackenprobe auch in erstarrtem Zustand umschließt und mit dieser aus der Sonde entnommen werden kann.

Zur Entnahme einer Probe wird die Sonde in das metallurgische Gefäß abgesenkt, bis sie im Bereich des Einlauftrichters 4 mit der Schlackenoberfläche in Berührung kommt. Der Querschnitt und die Geometrie des Einlauftrichters 4 ist in Abhängigkeit von der Dicke der Schlackenschicht 31 gewählt, so daß eine ausreichende Schlackenmenge in den Probenraum 2 eintreten kann, ohne daß Schmelze in den Probenraum eindringt. Bei einer besonders dünnen Schlackenschicht wird daher ein Einlauftrichter mit einem besonders großen Querschnitt gewählt, um auf die Substanz einer möglichst großen Schlackenfläche zurückgreifen zu können.

Sobald der Einlauftrichter 4 eine ausreichende Eintauchtiefe in die Stahlschmelze 32 erreicht hat, steigt die Schlacke aufgrund des ferrostatischen Drucks über den Speiser 3 in die Probenkammer 2, wobei die in dem Speiser und der Probenkammer 2 befindliche Luft über Entlüftungsöffnungen 6 entweicht. Wenn der Probenraum 2 mit Schlacke gefüllt ist, kommt die oberste Schlackenschicht mit der Abkühlplatte in Berührung, die bezüglich ihrer Dicke so dimensioniert ist, daß der Abkühlvorgang nicht zum Reißen oder Brechen der erstarrten Schlacke führt. Auch die Materialwahl der Abkühlplatte kann zu diesem Zwecke variiert werden. Der Speiser 3 mit seinem geringen Querschnitt verhindert einerseits ein Wiederauslaufen der Schlackenprobe und liefert eine Trennebene zwischen Einlauf und Probenkokille, so daß man konstante Probenabmessungen erhält.

Die nach der Probenentnahme im Probenraum 2 befindliche Schlackenprobe ist seitlich durch den Probenring 7 begrenzt, der die Schlackenprobe vollständig umgreift. Der Probenring 7 besitzt an seiner inneren, mit der Probe in Kontakt stehenden Fläche eine Oberflächenstruktur, die ein Herausfallen der Probe verhindert. Dies kann zum Beispiel durch Einkerbungen oder Riefen erreicht werden. Die Schlackenprobe kann nach dem Erstarren zusammen mit dem Probennng 7 aus der Sonde 1 entnommen werden. Die Abmessungen des Probenrings 7 sind so gewählt, daß dieser direkt in die Aufnahmemimik eines Analysegerätes eingesetzt werden kann. Aufgrund der Verwendung der Abkühlplatte 5 besitzt die Probe eine Oberfläche, die eine sofortige Analyse ohne vorherige Bearbeitung der Schlackenprobe erlaubt. Darüber hinaus ist die bruchanfällige Schlackenprobe beim Auspacken der Probe aus der Sonde und auch beim Transport geschützt, nämlich durch den Probenring 7.

Die oben beschriebene und in Fig. 1 dargestellte Schlackensonde erlaubt somit eine sichere Schlackenprobenentnahme, auch wenn die Sonde bis in die Schmelzenschicht eingetaucht wird, da aufgrund der geometrischen Verhältnisse des Einlauftrichters, des Schlackeneinlaufs und des Probenraumes ausschließlich Schlacke in den Probenraum eintreten kann. Durch Verwendung einer Saugpumpe kann darüber hinaus das Eintreten der Schlacke in den Probenraum unterstützt werden.

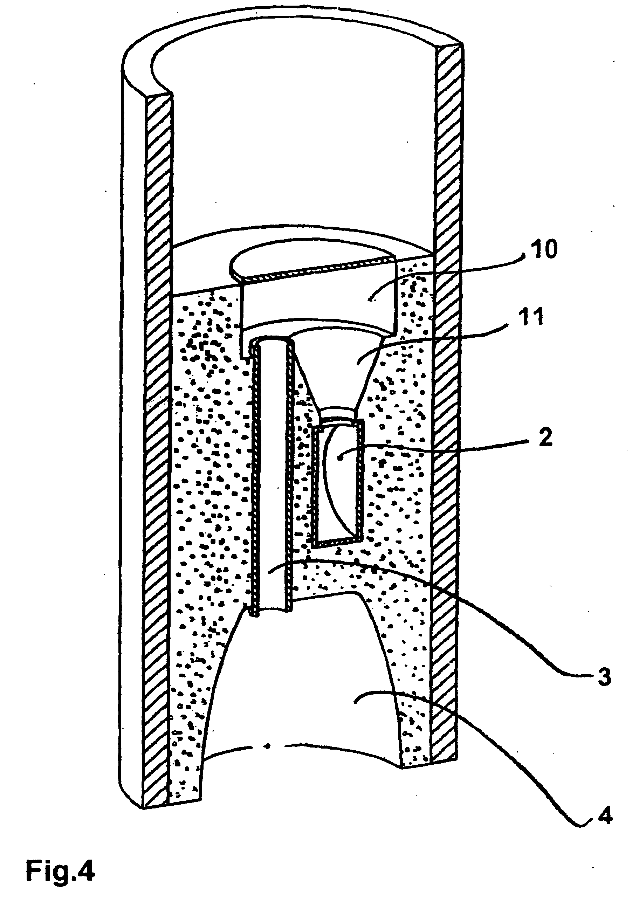

Die erfindungsgemäße Ausführungsform gemäß Fig. 4 besteht aus einer im Querschnitt kreisförmigen Sonde 1 mit einem Einlauftrichter 4, einem Speiser 3, einem Überlauf oder Überlaufkammer 10, einem Kokilleneinlauf 11 und einer Kokille bzw. Probenraum 2. Beim Eintauchen des Probennehmers in die Schlacke gelangt diese aufgrund des ferrostatischen Drucks über den Einlauftrichter 4 durch den Speiser 3 in den Überlauf 10 und der Schwerkraft folgend über den Kokilleneinlauf 11 in den Probenraum 2. Da der Kokilleneinlauf 11 oberhalb des Probenraums 2 angeordnet ist, füllt sich der Probenraum nach dem Eintauchen der Sonde in die Schlacke und verliert die Probe auch dann nicht, wenn die flüssige Schlacke aufgrund ihrer Viskosität beim Herausheben der Sonde aus der Schlacke aus dem Überlauf 10 und dem Steigrohr des Speisers 3 herausfließen sollte.

Die erfindungsgemäße Vorrichtung bzw. die beschriebenen Ausführungsformen sind auch in einer Kombisonde realisierbar, beispielsweise in Kombination mit einer Temperaturmessung und/oder einer EMK-Messung. Die erfindungsgemäße Vorrichtung kann dann auch in Funktion einer Sublanzensonde ausgebildet sein und lediglich eine von vielen Funktionen der Kombisonde ausüben.

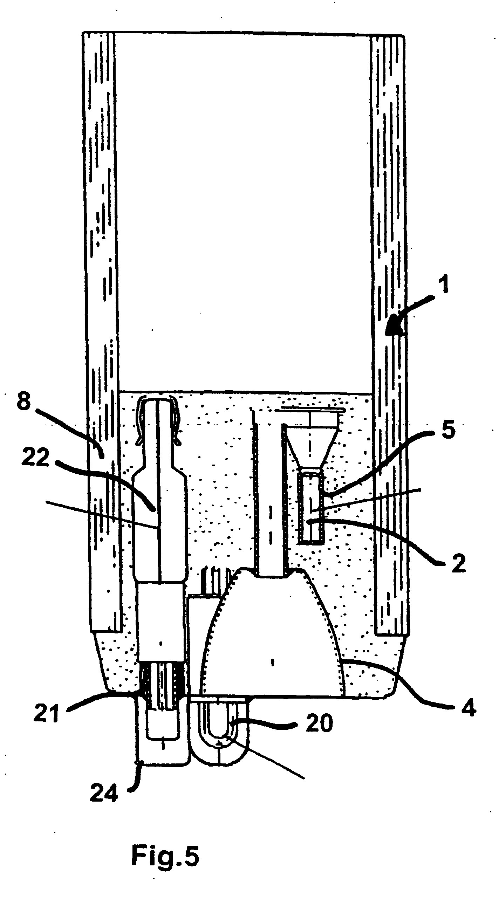

In Fig. 5 ist eine solche Kombisonde dargestein mit einem Thermoelement 20 und einem Stahlprobeneinlauf 21 mit der dazugehörigen Kokille 22. Thermoelement und Stahlprobeneinlauf sind durch Stahlkappen 23, 24 gegen die Schlackeneinwirkung beim Eintauchen geschützt.

Die Abkühlplatte besteht bei allen Ausführungsformen vorzugsweise aus Metall, kann aber alternativ aus Keramik, NE-Metall oder anderen Materialien bestehen.

## Patentansprüche

1. Vorrichtung zur Entnahme von Schlackenproben, mit einer Probenkokille (2), die einen Probenraum zur Aufnahme der zu analysierenden Schlacke aufweist, **dadurch gekennzeichnet, daß** sie einen Speiser (3) aufweist, durch den die Schlacke in die Vorrichtung geleitet wird, wobei der Speiser (3) derart gestaltet ist, daß die Schlacke beim Eintauchen der Vorrichtung in das flüssige Medium von unten nach oben in den Speiser (3) eintritt und mindestens in einem Punkt höher geführt wird als die Probenkokille (2).

2. Vorrichtung nach Anspruch 1, **dadurch gekennzeichnet, daß** der Einlauf des Speisers (3) unterhalb des Probenraums (2) der Probenkokille oder eines Überlaufs (10) angeordnet ist.

3. Vorrichtung nach einem der Ansprüche 1 oder 2, **dadurch gekennzeichnet, daß** der Speiser (3) einen geringeren Durchmesser als der Probenraum (2) der Probenkokille aufweist.

4. Vorrichtung nach Anspruch 2, **dadurch gekennzeichnet, daß** der Überlauf (10) in Form einer Überlaufkammer ausgebildet ist, die zwischen dem Speiser (3) und dem Probenraum (2) der Probenkokille angeordnet ist.

5. Vorrichtung nach einem oder mehreren der vorhergehenden Ansprüche, **dadurch gekennzeichnet, daß** mindestens eine Wandfläche des Probenraums (2) der Probenkokille als Abkühlplatte (5) ausgebildet ist.

6. Vorrichtung nach einem oder mehreren der vorhergehenden Ansprüche, **gekennzeichnet durch** einen unterhalb des Speisers (3) angeordneten Einlauftrichter (4).

7. Vorrichtung nach Anspruch 6 **dadurch gekennzeichnet, daß** das Volumen des Trichters (4) größer ist als das Volumen des Probenraumes (2) der Probenkokille.

8. Vorrichtung nach einem oder mehreren der vorhergehenden Ansprüche, **dadurch gekennzeichnet, daß** der Speiser (3) in einer Seitenwand der Vorrichtung neben dem Probenraum (2) der Probenkokille ausgebildet ist.

9. Vorrichtung nach einem oder mehreren der vorhergehenden Ansprüche, **dadurch gekennzeichnet, daß** eine entnehmbar im Probenraum (2) der Probenkokille angeordnete Probenfassung (7) die Seitenwände des Probenraumes (2) auskleidet.

10. Vorrichtung nach Anspruch 5, **dadurch gekennzeichnet, daß** die Abkühlplatte (5) aus metallischem oder keramischem Werkstoff besteht.

11. Vorrichtung nach Anspruch 10, **dadurch gekennzeichnet, daß** die Abkühlplatte (5) eine Dicke von maximal 2 mm aufweist.

12. Vorrichtung nach Anspruch 11, **dadurch gekennzeichnet, daß** die Abkühlplatte (5) eine Dicke von etwa 0,5 mm aufweist.

13. Vorrichtung nach Ansprunch 9, **dadurch gekennzeichnet, daß** die Probeneinfassung (7) mehrteilig ausgebildet ist.

14. Vorrichtung nach einem oder mehreren der Ansprüche 1 bis 13, **dadurch gekennzeichnet, daß** der Querschnitt des Probenraums größer ist als seine Dicke.

15. Vorrichtung nach einem oder mehreren der Ansprüche 1 bis 14, **dadurch gekennzeichnet, daß** der Einlauf (11) in die Probenkokille (2) oberhalb derselben angeordnet ist.

## Claims

1. Device for removal of slag samples, with a sample mould (2) having a sample chamber for accommodating the slag to be analysed, **characterised in that** it has a feeder (3) through which the slag is. fed into the device, whereby the feeder (3) is designed such that, on submerging of the device into the liquid medium, the slag enters the feeder (3) upwards from below and at least at one point is guided higher than the sample mould (2).

2. Device according to Claim 1, **characterised in that** the inlet of the feeder (3) is arranged below the sample chamber (2) of the sample mould or of an overflow (10).

3. Device according to one of the claims 1 or 2, **characterised in that** the feeder (3) has a smaller diameter than the sample chamber (2) of the sample mould.

4. Device according to Claim 2, **characterised in that** the overflow (10) is designed in the form of an overflow chamber arranged between the feeder (3) and the sample chamber (2) of the sample mould.

5. Device according to one or more of the previous claims, **characterised in that** at least one wall surface of the sample chamber (2) of the sample mould is designed as a cooling plate (5).

6. Device according to one or more of the previous claims, **characterised in that** it has an inlet funnel (4) arranged below the feeder (3).

7. Device according to Claim 6, **characterised in that** the volume of the funnel (4) is greater than the volume of the sample chamber (2) of the sample mould.

8. Device according to one or more of the previous claims, **characterised in that** the feeder (3) is formed in a side wall of the device adjacent to the sample chamber (2) of the sample mould.

9. Device according to one or more of the previous claims, **characterised in that** a removable sample holder (7) arranged in the sample chamber (2) of the sample mould lines the side walls of the sample chamber (2).

10. Device according to Claim 5, **characterised in that** the cooling plate (5) is made from a metallic or ceramic material.

11. Device according to Claim 10, **characterised in that** the cooling plate (5) has a thickness of no more than 2 mm.

12. Device according to Claim 11, **characterised in that** the cooling plate (5) has a thickness of approximately 0.5 mm.

13. Device according to Claim 9, **characterised in that** the sample holder (7) is formed from a plurality of parts.

14. Device according to one or more of the claims 1 to 13, **characterised in that** the cross-section of the sample chamber is greater than its thickness.

15. Device according to one or more of the claims 1 to 14, **characterised in that** the inlet (11) into the sample mould (2) is arranged above same.

## Revendications

1. Dispositif pour le prélèvement d'échantillons de laitier, avec une coquille à échantillon (2), présentant une chambre à échantillon pour recevoir le laitier à analyser, **caractérisé en ce qu'**il présente un alimentateur ou distributeur (3), au moyen duquel le laitier est dirigé dans le dispositif, l'alimentateur ou distributeur (3) étant réalisé de manière que, lors de l'immersion du dispositif dans le milieu liquide, le laitier pénètre du bas vers le haut dans l'alimentateur ou distributeur (3) et est guidé en au moins un point situé à un niveau plus haut que la coquille à échantillon (2).

2. Dispositif selon la revendication 1, **caractérisé en ce que** l'entrée de l'alimentateur (3) est disposée au-dessous de la chambre à échantillon (2) de la coquille à échantillon ou d'un trop-plein de déversement (10).

3. Dispositif selon l'une des revendication 1 ou 2, **caractérisé en ce que** l'alimentateur (3) présente un diamètre inférieur à celui de la chambre à échantillon (2) de la coquille à échantillon.

4. Dispositif selon la revendication 2, **caractérisé en ce que** le trop-plein de déversement (10) est réalisé sous la forme d'une chambre de déversement ou débordement, disposée entre l'alimentateur (3) et la chambre à échantillon (2) de la coquille à échantillon.

5. Dispositif selon l'une ou plusieurs des revendications précédentes, **caractérisé en ce qu'**au moins une surface de paroi de la chambre à échantillon (2) de la coquille à échantillon est réalisée sous la forme de plaque de refroidissement (5).

6. Dispositif selon l'une ou plusieurs des revendications précédentes, **caractérisé par** un entonnoir d'introduction (4), disposé au-dessous de l'alimentateur (3).

7. Dispositif selon la revendication 6, **caractérisé en ce que** le volume de l'entonnoir (4) est supérieur au volume de la chambre à échantillon (2) de la coquille à échantillon.

8. Dispositif selon l'une ou plusieurs des revendications précédentes, **caractérisé en ce que** l'alimentateur (3) est réalisé dans une paroi latérale du dispositif, à côté de la chambre à échantillon (2) de la coquille à échantillon.

9. Dispositif selon l'une ou plusieurs des revendications précédentes, **caractérisé en ce qu'**une bordure à échantillon (7), disposée de façon amovible dans la chambre à échantillon (2) de la coquille à échantillon, revêt les parois latérales de la chambre à échantillon (2).

10. Dispositif selon la revendication 5, **caractérisé en ce que** la plaque de refroidissement (5) es formée d'un matériau métallique ou céramique.

11. Dispositif selon la revendication 10, **caractérisé en ce que** la plaque de refroidissement (5) présente une épaisseur maximale de 2mm.

12. Dispositif selon la revendication 11, **caractérisé en ce que** la plaque de refroidissement (5) présente une épaisseur d'environ 0,5 mm.

13. Dispositif selon la revendication 9, **caractérisé en ce que** la bordure à échantillon (7) est réalisée en plusieurs parties.

14. Dispositif selon l'une ou plusieurs des revendications 1 à 13, **caractérisé en ce que** la largeur de la section transversale de la chambre à échantillon (7) est supérieure à son épaisseur.

15. Dispositif selon l'une ou plusieurs des revendications 1 à 14, **caractérisé en ce que** l'entrée (11) dans la coquille à échantillon (2) est disposée au-dessus de celle-ci.
